# EUROPEAN PATENT SPECIFICATION

(11) **EP 3 972 494 B1**
(45) Date of publication and mention of the grant of the patent: **25.09.2024**
(21) Application number: 20725547.2
(22) Date of filing: 14.05.2020
(51) Int. Cl.: A61B 6/00

(54) **COMBINED X-RAY SYSTEM AND PILOT TONE SYSTEM**
KOMBINIERTES RÖNTGENSYSTEM UND PILOTTONSYSTEM
SYSTÈME COMBINÉ DE RAYONS X ET SYSTÈME DE TONALITÉ DE PILOTE

(30) Priority: 20.05.2019 EP 19175305
(43) Date of publication of application: 30.03.2022
(73) Proprietor: Koninklijke Philips N.V., 5656 AG Eindhoven (NL)
(72) Inventor: LEUSSLER, Christoph, Guenther, 5656 AE Eindhoven (NL); FINDEKLEE, Christian, 5656 AE Eindhoven (NL); MEINEKE, Jan, Jakob, 5656 AE Eindhoven (NL); VERNICKEL, Peter, 5656 AE Eindhoven (NL); KOKEN, Peter, 5656 AE Eindhoven (NL)
(74) Representative: Philips Intellectual Property & Standards
(86) International application number: PCT/EP2020/063523
(87) International publication number: WO 2020/234124

(56) References cited:
- EP-A1- 3 413 076
- EP-A1- 3 413 076
- I GRAESSLIN ET AL: "Advancements in Contact-free Respiration Monitoring using RF Pick-up coils", PROCEEDINGS OF THE INTERNATIONAL SOCIETY FOR MAGNETIC RESONANCE IN MEDICINE, 1 January 2010 (2010-01-01), United States, pages 3045, XP055519013, Retrieved from the Internet <URL:https://cds.ismrm.org/protected/10MProceedings/files/3045_4702.pdf> [retrieved on 20181025], DOI: 10.1002/mrm.26866
- PFANNER FLORIAN ET AL: "Monitoring cardiac motion in CT using a continuous wave radar embedded in the patient table", MEDICAL PHYSICS, AIP, MELVILLE, NY, US, vol. 41, no. 8, XP012188955, ISSN: 0094-2405, [retrieved on 19010101], DOI: 10.1118/1.4886056
- PFANNER FLORIAN ET AL: "Monitoring cardiac motion in CT using a continuous wave radar embedded in the patient table", MEDICAL PHYSICS, AIP, MELVILLE, NY, US, vol. 41, no. 8, August 2014 (2014-08-01), XP012188955, ISSN: 0094-2405, [retrieved on 19010101], DOI: 10.1118/1.4886056
- I GRAESSLIN ET AL: "Advancements in Contact-free Respiration Monitoring using RF Pick-up coils", PROCEEDINGS OF THE INTERNATIONAL SOCIETY FOR MAGNETIC RESONANCE IN MEDICINE, 1 January 2010 (2010-01-01), United States, pages 3045, XP055519013, Retrieved from the Internet <URL:https://cds.ismrm.org/protected/10MProceedings/files/3045_4702.pdf> [retrieved on 20181025], DOI: 10.1002/mrm.26866

## Description

### FIELD OF THE INVENTION

The invention relates to X-ray imaging, in particular to the detection of subject motion using pilot tones.

### BACKGROUND OF THE INVENTION

In X-ray imaging techniques such as x-ray computed tomography, fluoroscopy, and X-ray imaging medical imaging data is acquired from a subject and used to reconstruct a medical image. This enables physicians or other health care professionals to accurately image a subject's internal anatomy. A disadvantage of these techniques is that the subject can move during the acquisition of the medical imaging data, which can lead to the addition of artifacts in the medical image or X-ray.

The European patent application EP 3 413 076 concerns an MRI system with a pilot tone generator for generating a cardiac movement signal. This known pilot tone emitter is formed as a separate single radio frequency source that generates a single pilot tone signal.

### SUMMARY OF THE INVENTION

The invention provides for an X-ray system, a computer program product and a method in the independent claims. Embodiments are given in the dependent claims.

Embodiments of the invention may provide for an improved method of determining the motion state of a subject for an X-ray system. Embodiments may provide this by using a pilot tone system. In a pilot tone system, a pilot tone signal (a radio frequency signal) is transmitted using a transmit coil and then pilot tone data is received using at least one receive coil. The pilot tone data is the signal in the receive coil caused by the transmission of the pilot tone signal. As the subject moves the RF coupling between the transmit coil and the receive coil changes. The change in the RF coupling causes a change in the pilot tone data which can be used to determine a motion state of the subject.

In one aspect the invention provides for an X-ray system configured for acquiring medical imaging data from a subject at least partially within an imaging zone. The X-ray system comprises a memory storing machine-executable instructions. The X-ray system further comprises a processor for controlling the X-ray system. The X-ray system further comprises a pilot tone system. The pilot tone system comprises a radio-frequency system comprising at least one transmit channel and at least one receive channel. The at least one transmit channel is configured for transmitting the at least one pilot tone signal via at least one transmit coil. The at least one receive channel is configured for receiving pilot tone data via at least one receive coil. The pilot tone system is based on transmission of the pilot tone signal as an electromagnetic signal e.g. in the radio frequency range of e.g. 40-400MHz. The pilot tone signal is transmitted in a continuous wave (cw) mode and the pilot tone data are due to impedance response to the transmitted pilot tone signal. This response is represented by the changes in amplitude and phase of the pilot tone data relative to that of the transmitted pilot tone signal. That is the pilot tone data represent a frequency domain response to the pilot tone signal and spectrally resolved information is carried by the pilot tone data.

Execution of the machine-executable instructions further cause the processor to transmit the at least one pilot tone signal, notably multi-channel pilot toen signals by controlling the at least one transmit channel. Execution of the machine-executable instructions further causes the processor to acquire the pilot tone data, notably multi-channel pilot tone data by controlling the at least one receive channel to receive the pilot tone data. Execution of the machine-executable instructions further causes the processor to determine a motion state of the subject using the pilot tone data. This embodiment may be beneficial because it may provide for an effective means of monitoring the motion state of the subject during an X-ray examination.

The X-ray system may take different forms in different examples. In one example the X-ray system is a computed tomography system or CT system. In another example the X-ray system is a fluoroscope.

In another embodiment the X-ray system is an X-ray imaging system that is configured for acquiring two-dimensional X-rays. All of the previously mentioned X-ray systems may benefit from the use of a pilot tone system.

In another embodiment the at least one transmit channel is multiple transmit channels. The at least one transmit coil has multiple transmit coils. The transmitting of the at least one pilot tone signal by controlling the at least one transmit channel comprises transmitting multi-channel pilot tone signals by controlling at least a portion of the multiple transmit channels. The acquisition of the pilot tone data by controlling the at least one receive channel to receive the pilot tone data comprises acquiring multi-channel pilot tone data by controlling at least a portion of the multiple receive channels to receive the pilot tone data.

The determination of a motion state of the subject using the pilot tone data comprises determining a motion state of the subject using the multi-channel pilot tone data. This embodiment may be beneficial because multiple transmit channels and multiple receive channels are used. This enables a larger degree of information on the motion of the subject.

The radio-frequency system is configured for encoding each of the multi-channel pilot tone signals using any one of the following: frequency encoding, phase encoding, complex modulation, CDMA encoding, and combinations thereof. This embodiment may be beneficial because it may provide for an effective means of differentiating the source of a pilot tone signal when receiving multiple signals. This may improve the ability to determine the motion state of the subject.

In another embodiment the at least one receive channel is multiple receive channels. The at least one receive coil is multiple receive coils. This embodiment may be beneficial because a number of receive coils may be placed in different locations and then the placement of the subject with respect to the pilot tone system is less sensitive.

In another embodiment the motion state is any one of the following: a subject motion location, a motion vector, a subject motion classification, a breathing state, a heart motion state, a translation vector descriptive of at least a portion of the subject, a rotation descriptive of at least a portion of the subject, and combinations thereof.

In another embodiment execution of the machine-executable instructions further causes the processor to determine the motion state using a recurrent neural network that is configured for receiving the pilot tone data and the at least one pilot tone signal and then for outputting the motion state. This embodiment may be beneficial because it may be trained to recognize complex patterns.

In another embodiment execution of the machine-executable instructions further causes the processor to determine the motion state by detecting a distance between the subject and each of the at least one receive coils. As a subject comes closer to a particular receive coil the coupling may increase. This may provide a model which may be used to efficiently yet simply determine the position of the subject.

In another embodiment execution of the machine-executable instructions further causes the processor to determine the motion state by detecting using digital filtering. Various periodic motion such as breathing or heartbeat will produce a pilot tone that has a characteristic frequency. The digital filtering enables the accurate and easy detection of such motion as heart or breathing.

In another embodiment execution of the machine-executable instructions further cause the processor to determine the motion state using principle component analysis. This embodiment may be beneficial because it may be possible to use large amounts of previously acquired pilot tone signals and pilot tone data to form the principle component analysis model.

In another embodiment execution of the machine-executable instructions further cause the processor to control the X-ray system to acquire the medical imaging data during acquisition of the pilot tone data. This embodiment may be beneficial because then the pilot tone data may be used for example controlling the function and operation of the X-ray system and/or later used for reconstructing an X-ray or medical image.

In another embodiment execution of the machine-executable instructions further cause the processor to reconstruct a medical image using the medical imaging data. Execution of the machine-executable instructions further cause the processor to correct the reconstruction of the medical image using the motion state of the subject. This embodiment may be particularly beneficial in cases where the X-ray system is a tomographic system such as a CT system. A knowledge of the position of the subject may help to account for motion of the subject during the reconstruction of the medical image.

In another embodiment execution of the machine-executable instructions further cause the processor to gate acquisition of the medical imaging data using the motion state of the subject. For example, if the motion state is a breathing phase or heart phase the gating of the subject may be useful for producing heart or breathing phase result images of the subject.

In another embodiment execution of the machine-executable instructions further causes the processor to modify acquisition of the medical imaging data using the motion state of the subject. For example, in a tomographic medical imaging technique such as CT the determination of the motion state may be used to change the style and position of the where the coordinate system for acquired medical imaging data is. This may for example be useful in compensating for motion of the subject during the acquisition of the medical imaging data.

In another embodiment the pilot tone system further comprises the at least one transmit coil and/or the at least one receive coil.

In another embodiment the X-ray system further comprises a subject support for supporting at least a portion of the subject in the imaging zone. The at least a portion of the at least one transmit coil and at least a portion of the at least one receive coil are integrated into the subject support. This may be beneficial because it may provide for an efficient means of incorporating the pilot tone system into the X-ray system.

The processor used to control the pilot tone system could be integrated into the subject support also. For example, the pilot tone system could be completely contained within the subject support. This could for example enable the addition of a pilot tone system to the X-ray system by the use of the subject support. The subject support could also be used for different imaging techniques such as magnetic resonance imaging. A single subject support could be moved to different imaging system as well as different types of imaging systems.

In another embodiment the X-ray system is an X-ray computed tomography system. This may be beneficial because the exact location of the subject may be beneficial in controlling the X-ray computed tomography system and/or during the reconstruction to improve the quality of medical images or X-rays from the X-ray system.

In another aspect the invention provides for a computer program product comprising machine-executable instructions for execution by a processor configured for controlling an X-ray system. The X-ray system is configured for acquiring medical imaging data from a subject at least partially within an imaging zone. The X-ray system comprises a pilot tone system. The pilot tone system comprises a radio-frequency system comprising at least one transmit channel and at least one receive channel. The at least one transmit channel is configured for transmitting at least one pilot tone signal via at least one transmit coil. The at least one receive channel is configured for receiving pilot tone data via at least one receive coil.

Execution of the machine-executable instructions further causes the processor to transmit the at least one pilot tone signal by controlling the at least one transmit channel. Execution of the machine-executable instructions further causes the processor to acquire the pilot tone data by controlling the at least one receive channel to receive the pilot tone data. Execution of the machine-executable instructions further causes the processor to determine a motion state of the subject using the pilot tone data.

In another aspect the invention provides for a method of operating an X-ray system configured for acquiring medical imaging data from a subject at least partially within an imaging zone. The X-ray system comprises a pilot tone system. The pilot tone system comprises a radio-frequency system comprising at least one transmit channel and at least one receive channel. The at least one transmit channel is configured for transmitting at least one pilot tone signal via at least one transmit coil. The at least one receive channel is configured for receiving pilot tone data via at least one receive coil. The method comprises transmitting the at least one pilot tone signal by controlling the at least one transmit channel. The method further comprises acquiring the pilot tone data by controlling the at least one receive channel to receive the pilot tone data. The method further comprises determining a motion state of the subject using the pilot tone data.

Disclosed herein if a magnetic resonance imaging system that comprises a memory storing machine-executable instructions and pulse sequence commands that are configured for controlling the magnetic resonance imaging system to acquire magnetic resonance imaging data. The magnetic resonance imaging system further comprises a processor for controlling the magnetic resonance imaging system.

The magnetic resonance imaging system further comprises a pilot tone system. The pilot tone system comprises a radio-frequency system comprising at least one transmit channel and at least one receive channel. The multiple receive channel is configured for receiving pilot tone data via the at least one transmit channel. Execution of the machine-executable instructions cause the processor to transmit the at least one pilot tone signal by controlling the at least one transmit channel. Execution of the machine-executable instructions further cause the processor to acquire pilot tone data by controlling the at least one receive channel to receive the pilot tone data. Execution of the machine-executable instructions further cause the processor to determine a motion state of the subject using the multi-channel pilot tone data.

Execution of the machine-executable instructions further cause the processor to determine a current gradient pulse frequency using the pulse sequence commands. Execution of the machine-executable instructions further cause the processor to detect subject motion with a periodicity within a predetermined range of the current gradient pulse frequency using the pilot tone data. Alternatively, subject motion may be detected by determining or detecting a correlation between the gradient pulse frequency and the pilot tone data. Execution of the machine-executable instructions further cause the processor to provide a peripheral nerve stimulation warning signal if the subject motion is detected.

The matching of the subject motion and the gradient pulse frequency could be determined in several different ways. In one case a frequency is determined from the periodicity of the gradient pulse waveform. This can be used to define a bandwidth where if the motion is detected using the pilot tone data then the peripheral nerve stimulation warning signal is provided. There may also be a threshold level or predetermined threshold level that is set to determine if the peripheral nerve stimulation is significant enough to warrant the providing of the warning signal.

The processor used to control the pilot tone system could be integrated into a subject support also. For example, the pilot tone system could be completely contained within the subject support and be used to add PNS detection to existing magnetic resonance imaging systems by using the subject support with the integrated pilot tone system.

Further disclosed is that execution of the machine-executable instructions further causes the processor to perform any one of the following if the peripheral nerve stimulation warning signal is provided: select alternative pulse sequence commands, modify the pulse sequence commands, and cancel execution of the pulse sequence commands. All of these alternatives may be beneficial because they may be used to reduce or eliminate the peripheral nerve stimulation. This may result in a higher degree of comfort for the subject in the magnetic resonance imaging system as well as reduce motion and thereby improve the quality of any resulting magnetic resonance image.

Further disclosed the magnetic resonance imaging system further comprises a magnetic resonance imaging coil. The magnetic resonance imaging coil comprises the at least one pilot tone transmit coil and/or the at least one receive coil. In various examples the coils can be integrated into the subject support of the magnetic resonance imaging system as well as being integrated into the magnetic resonance imaging coils or antennas that are used.

Further disclosed is that the magnetic resonance imaging system is configured for acquiring magnetic resonance imaging data within an imaging frequency range. The multiple transmit channels are configured for transmitting the unique pilot tone signals outside of the imaging frequency range. For example the pilot tone signals may be at a frequency higher than what is used for the magnetic resonance imaging. This may be beneficial because it may enable the simultaneous use of the magnetic resonance imaging system to acquire magnetic resonance imaging data as well as monitor subject motion using the pilot tone signals.

Further disclosed is that the at least one transmit channel is multiple transmit channels. It should be noted that all of the embodiments which were disclosed related to the multi-channel pilot tone signals and the multi-channel pilot tone data may also be applied to the magnetic resonance imaging system. For example there may be multiple transmit and receive channels.

Further disclosed is that the at least one receive channel is multiple receive channels. This may be particularly beneficial because the distance from the subject to the receive channels is very sensitive in determining the signal strength. If there are multiple receive channels then it is easier to properly place the subject such that a receive channel will receive a pilot tone signal which can be interpreted as subject motion.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a processor. `Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as two- or three-dimensional data that has been acquired using a medical imaging scanner such as a X-ray system. A medical imaging scanner is defined herein as an apparatus adapted for acquiring information about the physical structure of a patient and construct sets of two dimensional or three dimensional medical image data. Medical image data can be used to construct visualizations which are useful for diagnosis by a physician. This visualization can be performed using a computer.

Magnetic Resonance (MR) imaging data is defined herein as being the recorded measurements of radio frequency signals emitted by atomic spins using the antenna of a Magnetic resonance apparatus during a magnetic resonance imaging scan. Magnetic resonance data is an example of medical imaging data. A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of an X-ray system;
Fig. 2 shows a flow chart which illustrates a method of operating the X-ray system of Fig. 1;
Fig. 3 shows a flow chart which illustrates a further method of operating the X-ray system of Fig. 1;
Fig. 4 shows a flow chart which illustrates a further method of operating the X-ray system of Fig. 1;
Fig. 5 illustrates an example of multi-channel pilot tone data;
Fig. 6 illustrates a further example of an X-ray system;
Fig. 7 illustrates an example of a motion state determined using multi-channel pilot tone data;
Fig. 8 illustrates an example of a magnetic resonance imaging system;
Fig. 9 shows a flow chart which illustrates an example of a magnetic resonance imaging system; and
Fig. 10 illustrates an example of software system for a medical system.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

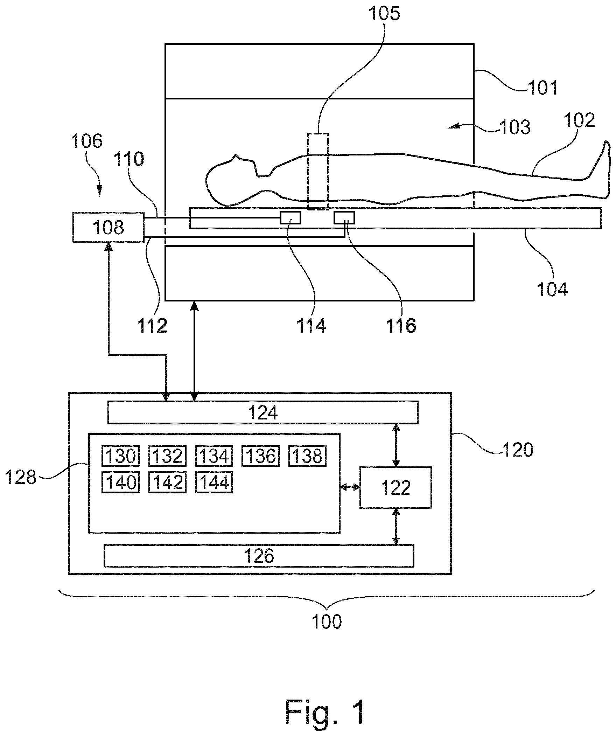

Fig. 1 illustrates an example of an X-ray system. In this example the X-ray system is a computed tomography system or CT system. However, this system depicted in Fig. 1 may also be for example a system for acquiring two-dimensional X-ray images such as a conventional X-ray system or a fluoroscope. The X-ray system 100 further comprises a computed tomography gantry 101 which has a bore 103. Within the bore is shown a subject 102 reposing on a subject support 104. The computed tomography gantry 101 has an imaging zone 105 where medical imaging data can be acquired.

The X-ray system 100 is shown as having a pilot tone system 106. The pilot tone system 106 comprises a radio-frequency system 108 that has at least one transmit channel 110 and at least one receive channel 112. The at least one transmit channel 110 is connected to at least one transmit channel 114. The at least one receive channel 112 is connected to at least one receive coil 116. In this example the at least one transmit coil 114 and the at least one receive coil 116 are built into the subject support 104. In other examples the coils 114, 116 could be placed in alternative locations such as supports surrounding the subject 102 or even in some instances adjacent or on the subject.

The X-ray system 100 is shown as further comprising a computer 120. The computer 120 comprises a processor 122. The processor 122 is intended to represent one or more processor or processing cores. The processor 122 could also be distributed amongst multiple computer systems 120. The processor 122 is shown as being connected to a hardware interface 124. The hardware interface 124 enables the processor 122 to send and receive commands and data to other components of the X-ray system 100. In some instances, the hardware interface 124 may for example be a network interface and enable the processor 122 to exchange data with other computer systems. The processor 122 is further shown as being connected to a user interface 126 and a memory 128.

The memory 128 may be any combination of memory which is accessible to the processor 122. This may include such things as main memory, cached memory, and also non-volatile memory such as flash RAM, hard drives, or other storage devices. In some examples the memory 128 may be considered to be a non-transitory computer-readable medium.

The memory 128 is shown as having machine-executable instructions 130. The machine-executable instructions 130 enable the processor 122 to control the operation and function of the X-ray system. The memory 128 is shown as containing control commands 132. The control commands may for instance be the specific commands for a particular imaging protocol to acquire medical imaging data 134. The memory 128 is shown as containing medical imaging data 134 that was acquired by controlling the X-ray system with the control commands 132. In some examples the control commands 132 may be incorporated into the machine-executable instructions 130. The memory 128 is further shown as containing a pilot tone signal 136 that may be transmitted using the at least one transmit channel 110.

The memory 128 is further shown as containing pilot tone data 138 that was received within the at least one receive channel 112 in response to transmitting the pilot tone signal 136. In some examples the pilot tone signal 136 may be multiple unique pilot tone signals 136 that may for example be encoded differently as was disclosed previously. The pilot tone data 138 may likewise be multi-channel pilot tone data. The memory 128 is further shown as containing a motion state 140 that was derived from at least the pilot tone data 138. The memory 128 may for example contain a motion state model 142.

The motion state model 142 may for example take the pilot tone 138 as input and optionally the pilot tone signal 136 to determine the motion state 140. The motion state model 142 may be implemented in different ways. In one example it may for example be a recurrent neural network, a convolutional neural network, a filter or other various models. The memory 128 is further shown as containing a medical image 144. The medical image 144 was reconstructed from the medical imaging data 134.

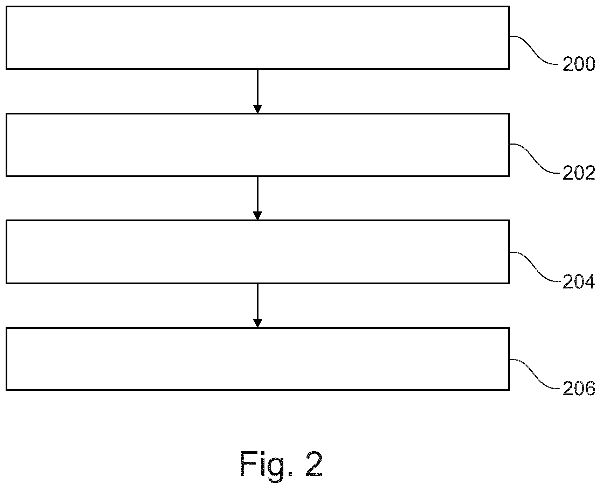

Fig. 2 shows a flowchart which illustrates a method of operating the X-ray system 100 of Fig. 1. First in step 200 the X-ray system is controlled to acquire the medical imaging data 134. As step 200 is performed steps 202, 204, and 206 are also performed. In step 202 the at least one pilot tone signal 136 is transmitted by controlling the at least one transmit channel 110. Next in step 204 the pilot tone data 138 is acquired by controlling the at least one receive channel 112. Finally, in step 206, the motion state 140 of the subject 102 is determined using the pilot tone data 138.

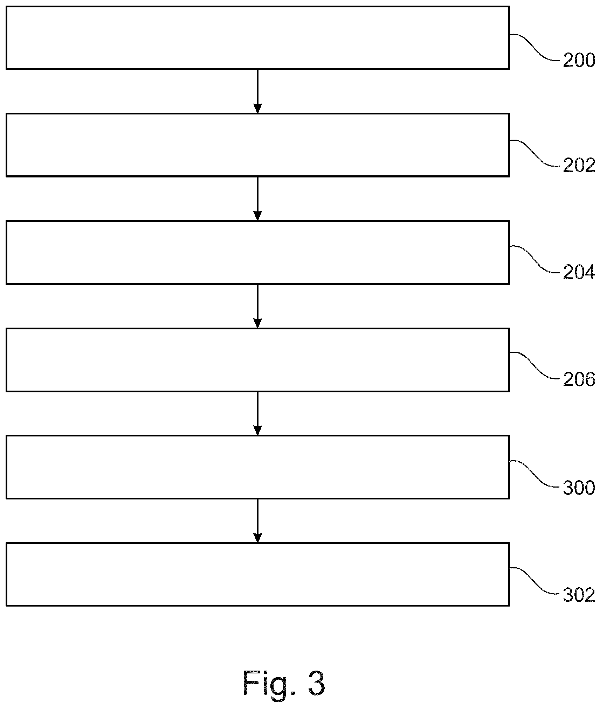

Fig. 3 illustrates an alternative method of operating the X-ray system 100 of Fig. 1. The method illustrated in Fig. 3 is similar to the method illustrated in Fig. 2 with the addition of several additional steps. Steps 200, 202, 204, 206 are performed as before. In this example step 206 may be performed after steps 200, 202, 204 are completely performed. Next in step 300 the medical image 144 is reconstructed using the medical imaging data 134. Finally, in step 302 the reconstruction of the medical image 144 is corrected using the motion state 140. This may for example be performed in different ways.

The motion state 140 could for example, if it is monitoring the heart or breathing phase, to divide the medical imaging data 134 into different bins and produce different images 144. In other examples if the motion state 140 is more detailed various portions of the medical imaging data 134 may be corrected and using during the reconstruction of the medical image 144. The method in Fig. 3 is an example of using the motion state 140 to perform retrospective correction of the medical image 144.

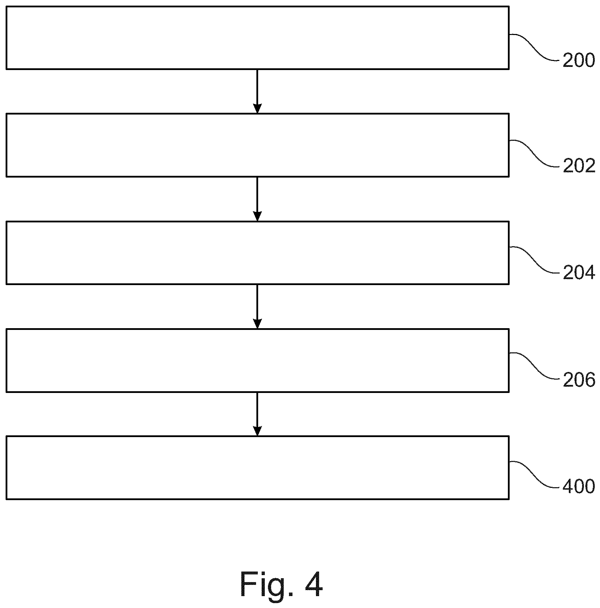

Fig. 4 illustrates a further example of a method of operating the X-ray system 100 of Fig. 1. The method in Fig. 4 is also similar to the method illustrated in Fig. 2. Steps 200, 202, 204, 206 are performed as before. In the example in Fig. 4 the determination of the motion state is done during the acquisition of the medical imaging data 134. In step 400 the medical imaging data is used to modify the acquisition of the medical imaging data. This could for example be used for changing the alignment or region which is imaged as well as to gate the acquisition of the medical imaging data.

Disclosed is a motion detection method, which may use distinct frequency, multi frequency or broadband signal sources for x-ray or computed tomography (CT) scanners to correct for motion and synchronize the scanner for cardiac imaging using multi-RF reference signals. The proposed method can substitute ECG triggering or the additional data is used to improve reconstruction, reduce radiation dose and improve workflow for autonomous imaging.

CT scans may use a number of input parameters and proper scan preparation. Depending on body size, body weight, patient position and anatomy to be scanned a protocol is chosen and modified to fit the patient. Typically, these data have to be entered manually. Physiology parameters (e.g. necessary for triggering scans or gating) are typically measured using dedicated sensors. It has been demonstrated recently, that relevant parameters can be deduced from live video-streams of a camera observing the patient during scanning. During a CT procedure the patient is covered by clothes.

Consequently, camera images are of limited use.

Pilot Tones are a contactless, electromagnetic navigators that offers monitoring of motion independently of the MR or CT acquisition. Generation and acquisition of Pilot Tone signals, can be done using, already existing, system integrated parts, such as MR local coils which would be used for magnetic resonance imaging.

Potential benefits and/or applications may include one or more of the following:
- ECG-free detection of heartbeats and breathing
- Separation and Quantification of Head - Body Motion
- Derive trigger for Cardiac and Respiratory Motion
- Applications for MR LINAC-Radiotherapy
- Pilot tone/ RF reference may be useful for accelerating patient preparation in order to increase quality and improve cost-effectiveness.
- provide a substitute for ECG electrodes
- reduce Extra workflow
- Reduce X-ray dose
- correct for or compensate for motion due to uncooperative subject
- provide feedback for patient
- Track subject motion through covers and clothes.

Examples may use pilot tone signals for X-Ray or CT scanner. In one example, the digital pilot signal antennas are located/integrated in the patient table.

For autonomous imaging using X-Ray the system may continuously monitor the 3D position. X-Radiation/imaging is triggered when there is no motion/movement detected or expected. The system allows body positioning during imaging or between imaging sequences or replacing a certain position.

For CT X-Ray beam is switched off during motion and system detects displacements from original position resulting in less dose.

In one example, the pilot tone signal may be monitored for active patient feedback. The patient/subject can reposition himself or the patient can align his position using a motion/position feedback monitor/sensor

In another example, the 3D/4D information from the pilot tone data may be included in the reconstruction.

In another example separate transmit and receive antennas and digital transmitters and receivers are used to allow to translate motion into complex 4 D datasets.

In another example, the data can also feed a convolution neuronal network or a recurrent neuronal network.

In another example, the pilot tone system is used with one or more additional motion detection system such as an optical camera, a radar, or ultrasonic acoustic detection.

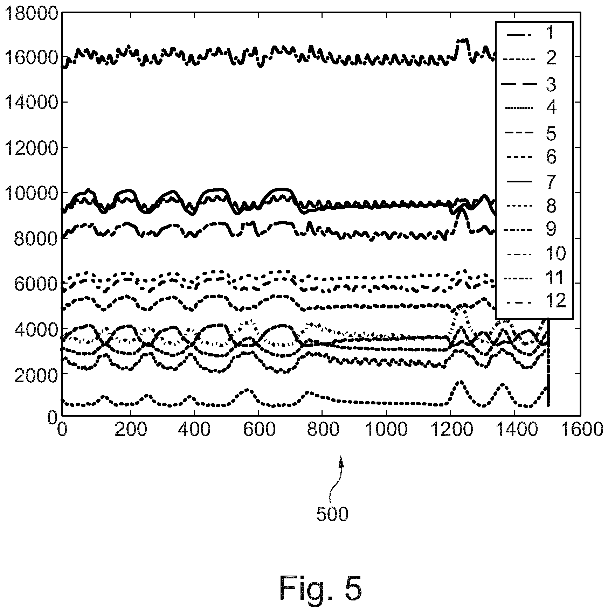

Fig. 5 illustrates an example of multi-channel pilot tone data 500. The pilot tone data 500 shown shows a number of plots of individual pilot tone signals that were measured. Cardiac signals and breathing motion are well detected, but strongly depends on individual antenna channel.

In examples, Local antennas (at least one receive coil 116) receive the narrow band signals (pilot tone signals). Each antenna feeds a preamplifier an individual software defined receiver. Complex signals are processed and the motion appears differently in the individual signals as changes in magnitude or phase. The data is further processed and the 3D/4D information is translated into motion parameters.

The data (pilot tone data) can also feed a convolution neuronal network or a recurrent neuronal network. A recurrent neural network (RNN) is a class of artificial neural network where connections between nodes form a directed graph along a sequence. This allows it to exhibit dynamic temporal behavior for a time sequence. Unlike feedforward neural networks, RNNs can use their internal state (memory) to process sequences of inputs (here different frequencies). This makes them applicable to tasks such as unsegmented, connected motion recognition or camera motion recognition.

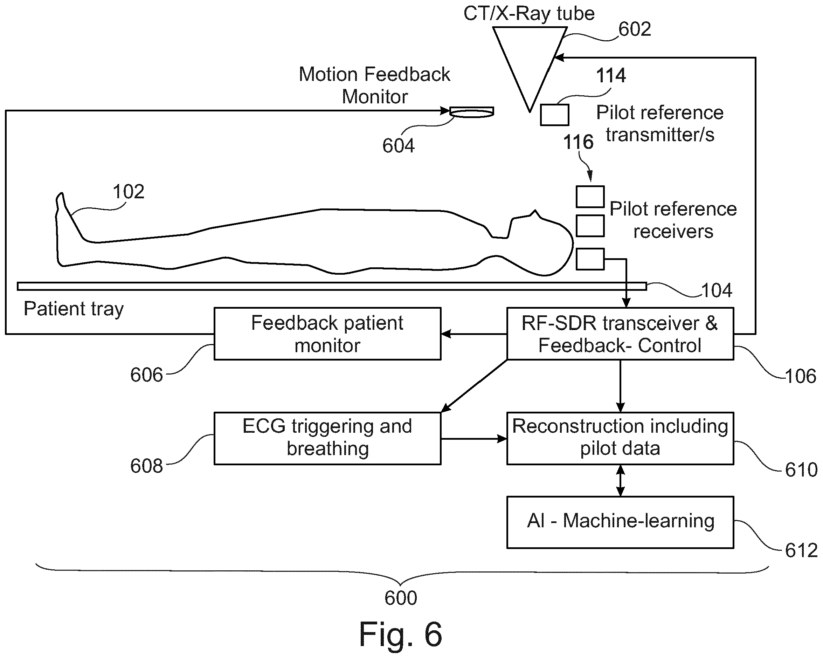

Fig. 6 illustrates a functional diagram of an X-ray system 600. In this example the X-ray system 600 comprises a CT or X-ray tube 602. Adjacent to a head of the subject 102 are a number of pilot reference receivers or multiple receive channels 116. Further away from the subject 102 is a single pilot reference transmitter which is a single transmit channel 110 or at least one transmit coil 114. There may also be multiple transmit coils 114 also. There is a supplementary motion feedback monitor 604. The motion feedback monitor 604 may be used to display to the subject 102 the current motion state and may help the subject 102 remain still. The X-ray system comprises an RF and SDR transceiver and feedback control which comprises the pilot tone system 106. This pilot tone system provides data to a feedback patient monitor 606. The feedback patient monitor 606 provides the image which is rendered by the motion feedback monitor 604. The pilot tone system 106 also provides data to ECG triggering and breathing triggering 608. The pilot tone system 106 also provides information to the reconstruction algorithm 610. An artificial or machine learning module 612 may be used for aiding during the reconstruction 610 as well as be trained with the data that is received via the pilot tone system 106.

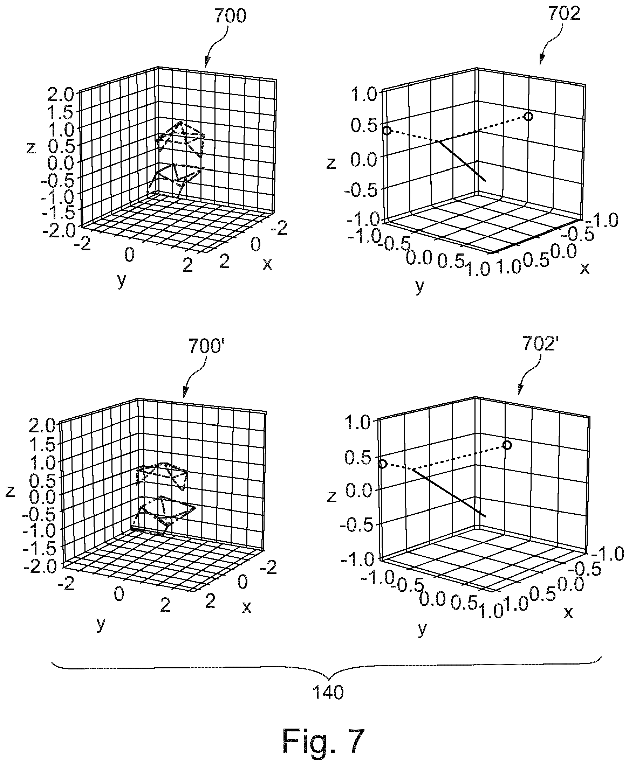

Fig. 7 illustrates examples of a motion state 140 which can be derived from multi-channel pilot tone data 500. Fig. 7 shows several position measurements that were used with multi-channel pilot tone data 500. In the examples illustrated in Fig. 7 a simple model was used to determine the distance of a subject's head from multiple receive coils 116. Plot 700 shows the position of a subject's head using this model. Plot 702 shows the direction of the subject's nose from the data in plot 700. Likewise plot 700' also shows a head position as measured using multiple receive coils. Plot 702' shows a change in the subject's nose orientation.

Fig 7 illustrates a simple example of how a set of pilot tone transmitters and receivers can be used to detect motion of the head. The images 702 and 702' show a very simple head model in form of the vector pointing form the back of the head to the tip of the nose in the real coordinate system. The origin of the coordinate system denotes the isocenter of the CT scanner. The back of the head is assumed to be fix to the patient table and cannot move.

The images 700 and 700' show a wire frame model of a 15-node pilot tone system. The positions of the nodes were derived from the spatial distribution of the pilot tone transmitters and receivers around the subject's head.

The coordinate system in these images is zeroed once the patient is placed on the patient table. Any movement will now affect the signal strength and phase among the different pilot tone transmitters and receivers. In the given model, a signal increase is mapped to the wire frame model by increasing the distance of the corresponding wire frame node from the (virtual) origin.

As a result, the wire frame gets distorted. The kind of distortion is characteristic for the head movement, e.g. shaking the head will lead to a signal increase for certain pilot tone transmitters and receiver combinations while others encounter a decrease. This insight was used for the simplified head model. The position of the nose tip is calculated by the spatially weighted average of the signals of the 15 pilot tone nodes. In the given example the volunteer turned his head from middle position (700, 702) to the right (negative y). In the wire frame model (700', 702'), this this resulted in a tilt-like distortion. Accordingly, the head vector model moves its tip to negative y.

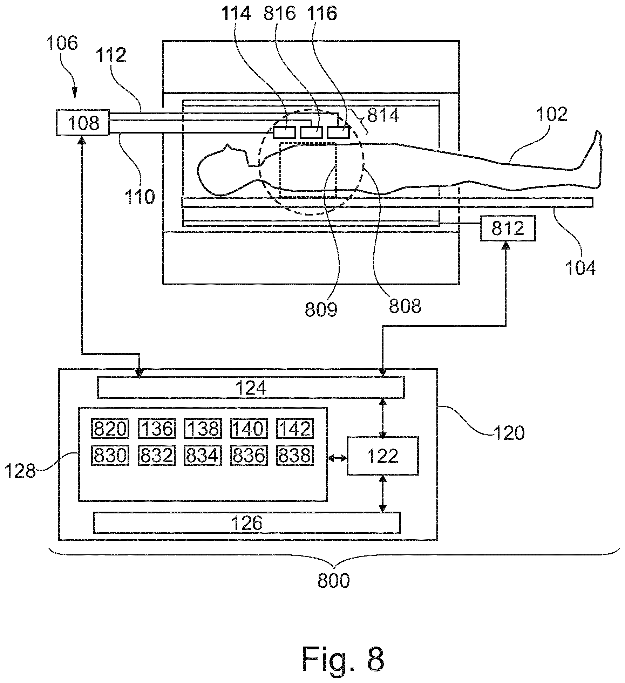

Fig. 8 illustrates an example of a magnetic resonance imaging system 800. Reference numerals reused in this figure indicate features or components which are equivalent to previously described features or components. Previously described components may not necessarily be described again.

The magnetic resonance imaging system 800 comprises a magnet 804. The magnet 804 is a superconducting cylindrical type magnet with a bore 806 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 806 of the cylindrical magnet 804 there is an imaging zone 808 where the magnetic field is strong and uniform enough to perform magnetic resonance imaging. A region of interest 809 is shown within the imaging zone 808. The magnetic resonance data that is acquired typically acquried for the region of interest. A subject 102 is shown as being supported by a subject support 104 such that at least a portion of the subject 102 is at least partially within the imaging zone 808 and the region of interest 809.

Within the bore 806 of the magnet there is also a set of magnetic field gradient coils 810 which is used for acquisition of preliminary magnetic resonance data to spatially encode magnetic spins within the imaging zone 808 of the magnet 804. The magnetic field gradient coils 810 connected to a magnetic field gradient coil power supply 812. The magnetic field gradient coils 810 are intended to be representative. Typically magnetic field gradient coils 810 contain three separate sets of coils for spatially encoding in three orthogonal spatial directions. A magnetic field gradient power supply supplies current to the magnetic field gradient coils. The current supplied to the magnetic field gradient coils 810 is controlled as a function of time and may be ramped or pulsed.

Within the bore 806 of the magnet 804 is a magnetic resonance imaging antenna 814. The magnetic resonance imaging antenna 814 is shown as comprising the multiple transmit coils 114 and the multiple receive coils 116. The magnetic resonance imaging antenna 814 also comprises a number of radio-frequency coils 816 which are used for performing the magnetic resonance imaging. The radio-frequency system 108 is also connected to the radio-frequency coil 816. The arrangement shown in Fig. 8 enables the acquisition of magnetic resonance imaging data simultaneous with the use of the pilot tone system. In other examples the radio-frequency coils 816 may also function as the multiple transceiver coils 114 and/or multiple receive coils 116.

The radio frequency coils 816 may also be referred to as a channel or antenna. The magnetic resonance antenna 814 is connected to a radio frequency system 108. The magnetic resonance antenna 814 and radio frequency system 108 may be replaced by separate transmit and receive coils and a separate transmitter and receiver. It is understood that the magnetic resonance antenna 814 and the radio frequency system 108 are representative. The magnetic resonance antenna 814 is intended to also represent a dedicated transmit antenna and a dedicated receive antenna. Likewise the system 816 may also represent a separate transmitter and receivers. The magnetic resonance antenna 814 may also have multiple receive/transmit elements and the radio frequency system 108 may have multiple receive/transmit channels. For example if a parallel imaging technique such as SENSE is performed, the radio-frequency system 108 could have multiple coil elements.

The radio frequency system 816 and the gradient controller 812 are shown as being connected to the hardware interface 124 of the computer system 128.

The memory 128 is shown as containing machine executable instructions 820. The machine executable instructions 820 enable the processor to control the magnetic resonance imaging system 800 as well as perform various data processing and image processing tasks. The memory 128 is further shown as containing pulse sequence commands 830 instead of control commands. The pulse sequence commands 830 are commands or data which may be converted into such commands which are used for controlling the operation of the magnetic resonance imaging system 800. The memory 128 is further shown as containing magnetic resonance imaging data 832 that was acquired by controlling the magnetic resonance imaging system with the pulse sequence commands 830.

The memory 128 is further shown as containing a magnetic resonance image 834 that was reconstructed from the magnetic resonance imaging data 832. The motion state 140 may be used in different ways. For example, the motion state 140 may be used for gating the acquisition of the magnetic resonance imaging data 832 as well as being used in the reconstruction of the magnetic resonance image 834.

The memory 128 may further contains a time-dependent gradient pulse frequency 836 that was determined from the pulse sequence commands 830. The motion state 140 may be compared with the time-dependent gradient pulse frequency 836 to determine if there is peripheral nerve stimulation in the subject 102. If the motion state correlates above a certain degree or above a certain amplitude within the same frequency range as motion detected, there may be a peripheral nerve stimulation warning signal 838 that is generated.

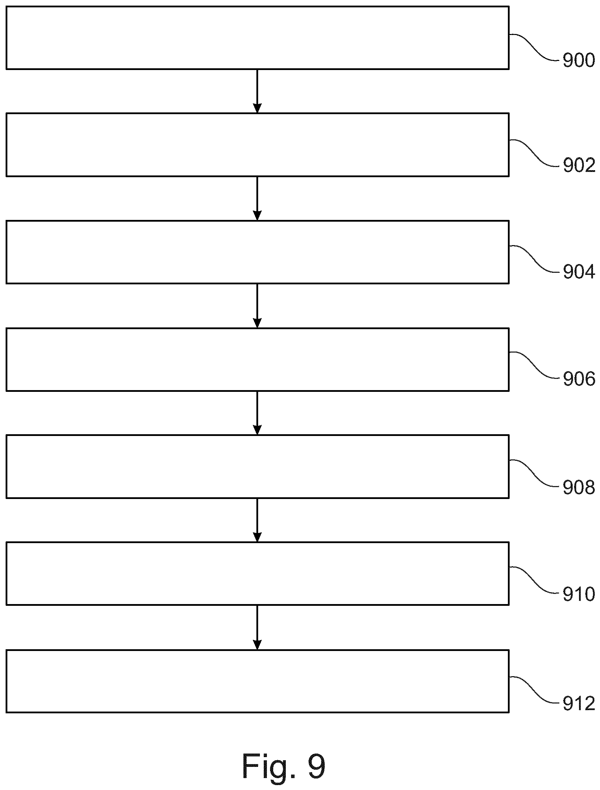

Fig. 9 shows a flowchart which illustrates a method of operating the magnetic resonance imaging system 800 of Fig. 8. First in step 900 the magnetic resonance imaging system 800 is controlled with the pulse sequence commands 832 to acquire the magnetic resonance imaging data 834. As step 900 is performed steps 902, 904, and 906 are also performed.

Next in step 902, the at least one pilot tone signals 136 are transmitted by controlling at least a portion of the multiple transmit channels 110. Next in step 904, the pilot tone data 138 is acquired by controlling the at least one receive channel 112.

Then in step 906, the motion state 140 of the subject 102 is determined using the pilot tone data 138. This could for example be performed using a recurrent neural network. In a recurrent neural network both the pilot tone data 138 and the pilot tone signals 136 could be input. In other cases, the motion state 140 can be determined from the multi-channel pilot tone data 138 alone. For example, the periodic breathing or heart motion of a subject 102 may cause the pilot tone data 138 to have a frequency component which is equal to or about equal to the heart rate and/or breathing rate. The heart and/or breathing motion may therefore be determined by the pilot tone data 138 alone.

In step 908, the time-dependent gradient pulse frequency 810 is determined using the pulse sequence commands 830. Next in step 910 subject motion with a periodicity within a predetermined range or correlation of the time-dependent gradient pulse frequency is detected using the motion state 140. For example, the motion state can be compared to the time-dependent gradient pulse frequency 810 or there can for example be a correlation that is calculated on the fly. Finally, in step 912 the peripheral nerve stimulation warning signal 812 is generated if the subject motion is detected.

Another application is the detection of Peripheral Nerve Stimulation during magnetic resonance imaging. It is possible to use the pilot tone signals acquired by the receive coil array and correlate it with gradient waveform signal to detect and trigger for PNS detection. The full matrix of the receive coil is measured and correlated with the gradient waveform to detect PNS.

If certain thresholds are reached, the MR sequence is adapted to reduce PNS. The sequence automatically adapts for patient comfortable parameters. Measure: change readout direction, change sequence, gradient strength, reposition patient. The data (multi-channel pilot tone data) can also feed a convolution neuronal network or a recurrent neuronal network.

Strong gradients applied during MRI exams can trigger peripheral nerve stimulation resulting in motion of muscle fibers or whole muscles.

### The PNS

Is discomfort for patient
level is individual for patient
Limits are set globally, disregarding individual sensitivity for PNS
Cannot be communicated by Patients with handicap or sedation. There is no quantitative feedback for operator,
cannot be detected by camera-based methods
Can induce MR artefact due to motion
Can lead to an unintended scan about, when the patient calls the operators due
PNS may be detected by using the pilot tone signals acquired by the receive coil array for PNS detection.

In general, PNS induced effects on the Pilot tone signals are expected to be lower than that of e.g. breathing. Due to this, and to distinguish from other motion the Pilot tone signals acquired by the receive coil may be correlated with the gradient waveform.

If certain thresholds are reached, the MR sequence is adapted to reduce PNS. The sequence automatically adapts for patient comfortable parameters. Possible measures are to change
change readout direction,
change sequence,
gradient strength,
position/pose of patient

Additional supplementary data may also be used such as optical, camera, radar, and ultrasonic acoustic detection.

Current MRI scanners feature a low-power transmit path independent from the transmit chain of the body coil for calibration purposes. Here, a small off-resonant coil is attached to the RF screen to the body coil. The transmit power for this coil was adjusted so that RF signals are in the same order of that originating from the spin system. Standard MRI coils are used for reception.

Pilot tone measurements can be interleaved or merged with the MR sequence. Tests showed that this setup allows to detect motion induced by breathing. Further tests were performed to increase the sensitivity of the set-up.

The Fig. 5 above shows an example of pilot tone magnitude signals. Additional information can be gained when simultaneously observing the phase of the acquired signals. The ideal position of the off-resonant coil was determined in tests to provide most sensitive outcome for breathing and heart motion. In the given experiments, the best setup was to place the coil on top of the patient's sternum. The acquisition of the pilot tone using all available RX coils allows for (limited) spatial sensitivity.

This insight can be used to distinguish different motion types.

It is likely that for PNS detection another position is more suitable, e.g., close to the long muscles of the patients back.

The data (multi-channel pilot tone data) can also feed a convolution neuronal network or a recurrent neuronal network. A recurrent neural network (RNN) is a class of artificial neural network where connections between nodes form a directed graph along a sequence. This allows it to exhibit dynamic temporal behavior for a time sequence. Unlike feedforward neural networks, RNNs can use their internal state (memory) to process sequences of inputs (here different frequencies). This makes them applicable to tasks such as unsegmented, connected motion recognition or camera motion recognition (see Fig13 below).

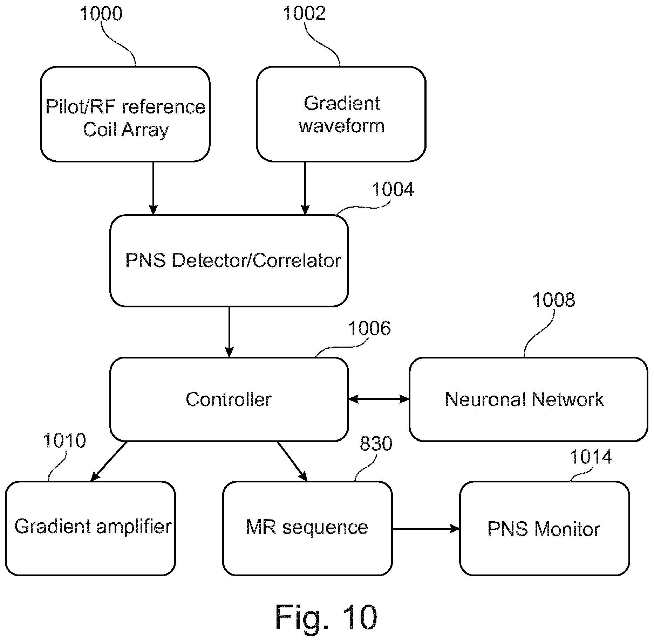

Fig. 10 illustrates a software algorithm and functional building blocks of a system that may for example be incorporated into a magnetic resonance imaging system such as the medical system 800 illustrated in Fig. 8. Block 1000 represents the pilot tone system and the radio-frequency reference coil array. Block 1002 represents the gradient waveform from the pulse sequence commands. Block 1004 represents a software component that is a peripheral nerve stimulation detector and/or correlator 1004. The detector or correlator 1004 is able to take information about the gradient waveform 1002 and information from the pilot tone data 1000 to detect if there is peripheral nerve stimulation. This is then fed into the controller 1006.

For example, the controller 1006 may be equivalent to the processor 122. This information could then be forwarded or processed from the controller and fed to a neural network 1008 that may for example be equivalent to a neural network. The controller 1006 can use a detection of the peripheral nerve stimulation for example to modify behavior of the gradient amplifier 1010, and possibly even modify the behavior or change the pulse sequence commands 832. This data may also be provided to a peripheral nerve stimulation monitor 1014. This for example may be provided via the user interface 126.

The following scheme illustrated in Fig. 10 shows how the Pilot Tone data may be processed and used.
- In a first step the Pilot Tone data is correlated with the gradient waveforms. Depending on the level of signal correlation the controller decides: correlation below first threshold = no low PNS: run the sequence as planned
- correlation below second threshold = considerable PNS: adapt sequence
- correlation above second threshold = PNS at pain limit or considerable image artefacts expected: terminate scan by gradient amplifier interlock

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: X-ray system
- 101: Computer Tomography Gantry
- 102: subject
- 103: bore
- 104: subject support
- 105: imaging zone
- 106: pilot tone system
- 108: radio frequency system
- 110: at least one transmit channel
- 112: at least one receive channel
- 114: at least one transmit coil
- 116: at least one receive coil
- 120: computer
- 122: processor
- 124: hardware interface
- 126: user interface
- 128: memory
- 130: machine executable instructions
- 132: control commands
- 134: medical imaging data
- 136: pilot tone signal
- 138: pilot tone data
- 140: motion state
- 142: motion state model
- 144: medical image
- 200: control the X-ray system to acquire the medical imaging data during acquisition of the pilot tone data
- 202: transmit the at least one pilot tone signal by controlling the at least one transmit channel
- 204: acquire the pilot tone data by controlling the at least one receive channel
- 206: determine a motion state of the subject using the pilot tone data
- 300: reconstruct a tomographic medical image using the medical imaging data
- 302: correct the reconstruction of the tomographic medical image using the motion state of the subject
- 400: gate acquisition of the medical imaging data using the motion state of the subject or modify acquisition of the medical imaging data using the motion state of the subject
- 500: multi-channel pilot tone data
- 600: X-ray system
- 602: CT or X-ray tube
- 604: motion feedback monitor
- 606: feedback patient monitor
- 608: ECG triggering and breating
- 610: reconstruction including pilot data
- 612: AI- machine learning module
- 700: head position
- 700': head position
- 702: nose position
- 702': nose position
- 800: magnetic resonance imaging system
- 804: magnet
- 806: bore of magnet
- 808: imaging zone
- 809: region of interest
- 810: magnetic field gradient coils
- 812: magnetic field gradient coil power supply
- 814: magnetic resonance antenna
- 816: radio-frequency coil
- 830: pulse sequence commands
- 832: magnetic resonance imaging data
- 834: magnetic resonance image
- 836: time dependent gradient pulse frequency
- 838: preripheral nerve stimulation warning signal
- 900: acquire magnetic resonance imaging data
- 902: transmit at least one pilot tone signal by controlling at least a portion of the multiple transmit channels to transmit the at least one pilot tone signal
- 904: acquire pilot tone data by controlling at least a portion of the multiple receive channels to receive the pilot tone data
- 906: determine a motion state of the subject using the pilot tone data
- 908: determine a current gradient pulse frequency using the pulse sequence commands
- 910: detect subject motion with a periodicity within a predetermined range of the current gradient pulse frequency using the pilot tone data
- 912: provide a peripheral nerve stimulation warning signal if the subject motion is detected
- 1000: Pilot/RF reference Coil Array
- 1002: Gradient Waveform
- 1004: PNS Detector/Correlator
- 1006: Controller
- 1008: Neuronal Network
- 1010: Gradient Amplifier
- 1014: PNS monitor

## Claims

1. An X-ray system (100, 700) configured for acquiring medical imaging data (134) from a subject (102) at least partially within an imaging zone (105), wherein the X-ray system comprises:
- a memory (128) storing machine executable instructions (130);
- a processor (122) configured for controlling the X-ray system; and
- a pilot tone system (106), **characterized in that** the pilot tone system comprises a radio frequency system (108) comprising multiple transmit channels (110) and multiple receive channel (112), wherein the multiple transmit channels are configured for transmitting multi-channel pilot tone signals (136) via multiple transmit coils (114); wherein the multiple receive channels are configured for receiving multi-channel pilot tone data (138) via multiple receive coils (116);
wherein execution of the machine executable instructions causes the processor to:
- transmit (202) the multi-channel pilot tone signals by controlling the multiple transmit channels;
- acquire (204) the multi-channel pilot tone data by controlling the multiple receive channels; and
- determine (206) a motion state (140, 700, 700', 702, 702') of the subject using the multi-channel pilot tone data.

2. The X-ray system of claim 1, wherein the radio frequency system is configured for encoding each of the multi-channel pilot tone signals using any one of the following: frequency encoding, phase encoding, complex modulating, CDMA encoding, and combinations thereof.

3. The X-ray system of any one of claims 1 through 2, wherein the motion state is any one of the following:
- subject motion location (700, 700', 702, 702');
- a motion vector;
- a subject motion classification;
- a breathing state;
- a heart motion state;
- a translation vector descriptive of at least a portion of the subject;
- a rotation descriptive of at least a portion of the subject; and
- combinations thereof.

4. The X-ray system of any one of claims 1 through 3, wherein execution of the machine executable instructions causes the processor to determine the motion state using any one of the following:
- using a recurrent neural network configured for receiving the multi-channel pilot tone data and the at least one multi-channel pilot tone signals and for outputting the motion state;
- detecting a distance between the subject and each of the at least one receive coils;
- using digital filtering;
- using principal component analysis; and
- combinations thereof.

5. The X-ray system of any one of the preceding claims,
where execution of the machine executable instructions further causes the processor to control (200) the X-ray system to acquire the medical imaging data during acquisition of the pilot tone data.

6. The X-ray system of any one of the preceding claims, wherein execution of the machine executable instructions further causer the processor to:
- reconstruct (300) a medical image (144) using the medical imaging data; and
- correct (302) the reconstruction of the tomographic medical image using the motion state of the subject.

7. The X-ray system of claim 5 or 6, wherein execution of the machine executable instructions further causes the processor to gate (400) acquisition of the medical imaging data using the motion state of the subject.

8. The X-ray system of claim 5, 6, or 7, wherein execution of the machine executable instructions further causes the processor to modify (400) acquisition of the medical imaging data using the motion state of the subject.

9. The X-ray system of any one of the preceding claims, wherein the pilot tone system further comprises the multiple transmit coils and/or the multiple receive coil.

10. The medical system of claim 9, wherein the X-ray comprises a subject support (104) for supporting at least a portion of the subject in the imaging zone, wherein the at least a portion of the multpile transmit coils and at least a portion of the multiple receive coils are integrated into the subject support.

11. The medical system of any one of the preceding claims, wherein the X-ray system is an X-ray computed tomography system.

12. A computer program product comprising machine executable instructions for execution by a processor (122) configured for controlling an X-ray system (100, 700), wherein the X-ray system is configured for acquiring medical imaging data (134) from a subject (102) at least partially within an imaging zone (105), wherein the X-ray system comprises a pilot tone system (106), wherein the pilot tone system comprises a radio frequency system (108) comprising multiple transmit channels (110) and multiple receive channels (112) , wherein the multiple transmit channels are configured for transmitting multiple multi-channel pilot tone signals (136) via multiple transmit coils (114), wherein the multiple receive channels are configured for receiving multi-channel pilot tone data (138) via multiple receive coils (116), wherein execution of the machine executable instructions causes the processor to:
- transmit (202) the multiple multi-channel pilot tone signals by controlling the multiple transmit channels;
- acquire (204) the multi-channel pilot tone data by controlling the multiple receive channels to receive the multi-channel pilot tone data; and
- determine (206) a motion state (140, 700, 700', 702, 702') of the subject using the multi-channel pilot tone data.

13. A method of operating an X-ray system (100, 700) configured for acquiring medical imaging data (134) from a subject (102) at least partially within an imaging zone (105), wherein the X-ray system comprises a pilot tone system (106), wherein the pilot tone system comprises a radio frequency system (108) comprising multiple transmit channels (110) and multiple receive channels (112), wherein the multiple transmit channels are configured for transmitting multiple multi-channel pilot tone signals via multiple transmit coils (114), wherein the multiple receive channels are configured for receiving multi-channel pilot tone data via multiple receive coils (116), wherein the method comprises:
- transmitting (202) the multiple multi-channel pilot tone signals by controlling the multiple transmit channels;
- acquiring (204) the multi-channel pilot tone data by controlling the multiple receive channels to receive the multi-channel pilot tone data; and
- determining (206) a motion state (140, 700, 700', 702, 702') of the subject using the multi-channel pilot tone data.

## Patentansprüche

1. Röntgensystem (100, 700), das zum Erfassen medizinischer Bildgebungsdaten (134) von einer Person (102) konfiguriert ist, die sich zumindest teilweise innerhalb einer Bildgebungszone (105) befindet, wobei das Röntgensystem umfasst:
- einen Speicher (128), der maschinenausführbare Anweisungen (130) speichert;
- einen Prozessor (122), der zum Steuern des Röntgensystems konfiguriert ist; und
- ein Pilottonsystem (106), **dadurch gekennzeichnet, dass** das Pilottonsystem ein Hochfrequenzsystem (108) umfasst, das mehrere Sendekanäle (110) und mehrere Empfangskanäle (112) umfasst, wobei die mehreren Sendekanäle zum Senden von Mehrkanal-Pilottonsignalen (136) über mehrere Sendespulen (114) konfiguriert sind; wobei die mehreren Empfangskanäle zum Empfangen von Mehrkanal-Pilottondaten (138) über mehrere Empfangsspulen (116) konfiguriert sind;
wobei die Ausführung der maschinenausführbaren Anweisungen bewirkt, dass der Prozessor:
- die Mehrkanal-Pilottonsignale durch Steuern der mehreren Sendekanäle sendet (202);
- die Mehrkanal-Pilottondaten durch Steuern der mehreren Empfangskanäle erfasst (204); und
- einen Bewegungszustand (140, 700, 700', 702, 702') der Person unter Verwendung der Mehrkanal-Pilottondaten bestimmt (206).

2. Röntgensystem nach Anspruch 1, wobei das Hochfrequenzsystem zum Codieren jedes der Mehrkanal-Pilottonsignale unter Verwendung eines von Folgendem konfiguriert ist: Frequenzcodierung, Phasencodierung, komplexe Modulation, CDMA-Codierung und Kombinationen davon.

3. Röntgensystem nach einem der Ansprüche 1 bis 2, wobei der Bewegungszustand einer von Folgenden ist:
- Bewegungsstelle der Person (700, 700', 702, 702');
- ein Bewegungsvektor;
- eine Bewegungsklassifizierung der Person;
- ein Atemzustand;
- ein Herzbewegungszustand;
- ein Translationsvektor, der zumindest einen Abschnitt der Person beschreibt;
- eine Rotation, die zumindest einen Abschnitt der Person beschreibt; und
- Kombinationen davon.

4. Röntgensystem nach einem der Ansprüche 1 bis 3, wobei die Ausführung der maschinenausführbaren Anweisungen bewirkt, dass der Prozessor den Bewegungszustand unter Verwendung eines von Folgendem bestimmt:
- Verwenden eines rekurrierenden neuronalen Netzwerks, das zum Empfangen der Mehrkanal-Pilottondaten und des mindestens einen Mehrkanal-Pilottonsignals und zum Ausgeben des Bewegungszustands konfiguriert ist;
- Erfassen einer Distanz zwischen der Person und jeder der mindestens einen Empfangsspulen;
- Verwendung digitaler Filterung;
- Verwenden von Hauptkomponentenanalyse; und
- Kombinationen davon.

5. Röntgensystem nach einem der vorstehenden Ansprüche,
wobei die Ausführung der maschinenausführbaren Anweisungen weiter bewirkt, dass der Prozessor das Röntgensystem steuert (200), um die medizinischen Bildgebungsdaten bei der Erfassung der Pilottondaten zu erfassen.

6. Röntgensystem nach einem der vorstehenden Ansprüche, wobei die Ausführung der maschinenausführbaren Anweisungen weiter bewirkt, dass der Prozessor:
- ein medizinisches Bild (144) unter Verwendung der medizinischen Bildgebungsdaten rekonstruiert (300); und
- die Rekonstruktion des tomographischen medizinischen Bildes unter Verwendung des Bewegungszustandes der Person korrigiert (302).

7. Röntgensystem nach Anspruch 5 oder 6, wobei die Ausführung der maschinenausführbaren Anweisungen weiter bewirkt, dass der Prozessor die medizinischen Bildgebungsdaten unter Verwendung des Bewegungszustands der Person ansteuert (400).

8. Röntgensystem nach Anspruch 5, 6 oder 7, wobei die Ausführung der maschinenausführbaren Anweisungen weiter bewirkt, dass der Prozessor die medizinischen Bildgebungsdaten unter Verwendung des Bewegungszustands der Person ändert (400).

9. Röntgensystem nach einem der vorstehenden Ansprüche, wobei das Pilottonsystem weiter die mehreren Sendespulen und/oder die mehreren Empfangsspulen umfasst.

10. Medizinisches System nach Anspruch 9, wobei das Röntgengerät einen Personenträger (104) zum Tragen von zumindest einem Abschnitt der Person in der Bildgebungszone umfasst, wobei der zumindest eine Abschnitt der mehreren Sendespulen und der zumindest eine Abschnitt der mehreren Empfangsspulen in dem Personenträger integriert sind.

11. Medizinisches System nach einem der vorstehenden Ansprüche, wobei das Röntgensystem ein Röntgen-Computertomographiesystem ist.

12. Computerprogrammprodukt, welches maschinenausführbare Anweisungen umfasst zum
Ausführen durch einen Prozessor (122), der zum Steuern eines Röntgensystems (100, 700) konfiguriert ist, wobei das Röntgensystem zum Erfassen medizinischer Bildgebungsdaten (134) von einer Person (102) konfiguriert ist, die sich zumindest teilweise innerhalb einer Bildgebungszone (105) befindet, wobei das Röntgensystem ein Pilottonsystem (106) umfasst, wobei das Pilottonsystem ein Hochfrequenzsystem (108) umfasst, das mehrere Sendekanäle (110) und mehrere Empfangskanäle (112) umfasst, wobei die mehreren Sendekanäle zum Senden mehrerer Mehrkanal-Pilottonsignale (136) über mehrere Sendespulen (114) konfiguriert sind, wobei die mehreren Empfangskanäle zum Empfangen von Mehrkanal-Pilottondaten (138) über mehrere Empfangsspulen (116) konfiguriert sind, wobei die Ausführung der maschinenausführbaren Anweisungen weiter bewirkt, dass der Prozessor:
- die mehreren Mehrkanal-Pilottonsignale durch Steuern der mehreren Sendekanäle sendet (202);
- die Mehrkanal-Pilottondaten durch Steuern der mehreren Empfangskanäle erfasst (204), um die Mehrkanal-Pilottondaten zu empfangen; und
- einen Bewegungszustand (140, 700, 700', 702, 702') der Person unter Verwendung der Mehrkanal-Pilottondaten bestimmt (206).

13. Verfahren zum Betreiben eines Röntgensystems (100, 700), das zum Erfassen medizinischer Bildgebungsdaten (134) von einer Person (102) konfiguriert ist, die sich zumindest teilweise innerhalb einer Bildgebungszone (105) befindet, wobei das Röntgensystem ein Pilottonsystem (106) umfasst, wobei das Pilottonsystem ein Hochfrequenzsystem (108) umfasst, das mehrere Sendekanäle (110) und mehrere Empfangskanäle (112) umfasst, wobei die mehreren Sendekanäle zum Senden mehrerer Mehrkanal-Pilottonsignale über mehrere Sendespulen (114) konfiguriert sind, wobei die mehreren Empfangskanäle zum Empfangen von Mehrkanal-Pilottondaten über mehrere Empfangsspulen (116) konfiguriert sind, wobei das Verfahren umfasst:
Senden (202) der mehreren Mehrkanal-Pilottonsignale durch Steuern der mehreren Sendekanäle;
- Erfassen (204) der Mehrkanal-Pilottondaten durch Steuern der mehreren Empfangskanäle, um die Mehrkanal-Pilottondaten zu empfangen; und
- Bestimmen (206) eines Bewegungszustands (140, 700, 700', 702, 702') der Person unter Verwendung der Mehrkanal-Pilottondaten.

## Revendications

1. Système à rayons X (100, 700) configuré pour acquérir des données d'imagerie médicale (134) d'un sujet (102) au moins partiellement à l'intérieur d'une zone d'imagerie (105), dans lequel le système à rayons X comprend :
- une mémoire (128) stockant des instructions exécutables par machine (130) ;
- un processeur (122) configuré pour commander le système à rayons X ; et
- un système de tonalité pilote (106), **caractérisé en ce que** le système de tonalité pilote comprend un système radiofréquence (108) comprenant de multiples canaux d'émission (110) et de multiples canaux de réception (112), dans lequel les multiples canaux d'émission sont configurés pour émettre des signaux de tonalité pilote multicanaux (136) via de multiples bobines d'émission (114) ; dans lequel les multiples canaux de réception sont configurés pour recevoir des données de tonalité pilote multicanaux (138) via de multiples bobines de réception (116) ;
dans lequel l'exécution des instructions exécutables par machine amène le processeur à :
- émettre (202) les signaux de tonalité pilote multicanaux par commande des multiples canaux d'émission ;
- acquérir (204) les données de tonalité pilote multicanaux par commande des multiples canaux de réception ; et
- déterminer (206) un état de mouvement (140, 700, 700', 702, 702') du sujet en utilisant les données de tonalité pilote multicanaux.

2. Système à rayons X selon la revendication 1, dans lequel le système radiofréquence est configuré pour coder chacun des signaux de tonalité pilote multicanaux en utilisant l'un quelconque des suivants : un codage de fréquence, un codage de phase, une modulation complexe, un codage CDMA et des combinaisons de ceux-ci.

3. Système à rayons X selon l'une quelconque des revendications 1 à 2, dans lequel l'état de mouvement est l'un quelconque des suivants :
- un emplacement de mouvement de sujet (700, 700', 702, 702') ;
- un vecteur de mouvement ;
- une classification de mouvement de sujet ;
- un état respiratoire ;
- un état de mouvement cardiaque ;
- un vecteur de translation décrivant au moins une partie du sujet ;
- une rotation décrivant au moins une partie du sujet ; et
- des combinaisons de ceux-ci.

4. Système à rayons X selon l'une quelconque des revendications 1 à 3, dans lequel l'exécution des instructions exécutables par machine amène le processeur à déterminer l'état de mouvement en utilisant l'un quelconque des suivants :
- l'utilisation d'un réseau neuronal récurrent configuré pour recevoir les données de tonalité pilote multicanaux et les au moins un signaux de tonalité pilote multicanaux et pour délivrer l'état de mouvement ;
- la détection d'une distance entre le sujet et chacune des au moins une bobines de réception ;
- l'utilisation d'un filtrage numérique ;
- l'utilisation d'une analyse en composantes principales ; et
- des combinaisons de ceux-ci.

5. Système à rayons X selon l'une quelconque des revendications précédentes,
dans lequel l'exécution des instructions exécutables par machine amène en outre le processeur à commander (200) le système à rayons X pour acquérir les données d'imagerie médicale pendant l'acquisition des données de tonalité pilote.

6. Système à rayons X selon l'une quelconque des revendications précédentes, dans lequel l'exécution des instructions exécutables par machine amène en outre le processeur à :
- reconstruire (300) une image médicale (144) en utilisant les données d'imagerie médicale ; et
- corriger (302) la reconstruction de l'image médicale tomographique en utilisant l'état de mouvement du sujet.

7. Système à rayons X selon la revendication 5 ou 6, dans lequel l'exécution des instructions exécutables par machine amène en outre le processeur à déclencher (400) l'acquisition des données d'imagerie médicale en utilisant l'état de mouvement du sujet.

8. Système à rayons X selon la revendication 5, 6 ou 7, dans lequel l'exécution des instructions exécutables par machine amène en outre le processeur à modifier (400) l'acquisition des données d'imagerie médicale en utilisant l'état de mouvement du sujet.

9. Système à rayons X selon l'une quelconque des revendications précédentes, dans lequel le système de tonalité pilote comprend en outre les multiples bobines d'émission et/ou les multiples bobines de réception.

10. Système médical selon la revendication 9, dans lequel les rayons X comprennent un support de sujet (104) pour supporter au moins une partie du sujet dans la zone d'imagerie, dans lequel les au moins une partie des multiples bobines d'émission et au moins une partie des multiples bobines de réception sont intégrées au support de sujet.

11. Système médical selon l'une quelconque des revendications précédentes, dans lequel le système à rayons X est un système de tomodensitométrie à rayons X.

12. Produit de programme informatique comprenant des instructions exécutables par machine pour l'exécution par un processeur (122) configuré pour commander un système à rayons X (100, 700), dans lequel le système à rayons X est configuré pour acquérir des données d'imagerie médicale (134) d'un sujet (102) au moins partiellement dans une zone d'imagerie (105), dans lequel le système à rayons X comprend un système de tonalité pilote (106), dans lequel le système de tonalité pilote comprend un système radiofréquence (108) comprenant de multiples canaux d'émission (110) et de multiples canaux de réception (112), dans lequel les multiples les canaux d'émission sont configurés pour émettre de multiples signaux de tonalité pilote multicanaux (136) via de multiples bobines d'émission (114), dans lequel les multiples canaux de réception sont configurés pour recevoir des données de tonalité pilote multicanaux (138) via de multiples bobines de réception (116), dans lequel l'exécution des instructions exécutables par machine amène le processeur à :
- émettre (202) les multiples signaux de tonalité pilote multicanaux par commande des multiples canaux d'émission ;
- acquérir (204) les données de tonalité pilote multicanaux par commande des multiples canaux de réception pour recevoir les données de tonalité pilote multicanaux ; et
- déterminer (206) un état de mouvement (140, 700, 700', 702, 702') du sujet en utilisant les données de tonalité pilote multicanaux.

13. Procédé de fonctionnement d'un système à rayons X (100, 700) configuré pour acquérir des données d'imagerie médicale (134) d'un sujet (102) au moins partiellement dans une zone d'imagerie (105), dans lequel le système à rayons X comprend un système de tonalité pilote (106), dans lequel le système de tonalité pilote comprend un système radiofréquence (108) comprenant de multiples canaux d'émission (110) et de multiples canaux de réception (112), dans lequel les multiples canaux d'émission sont configurés pour émettre de multiples signaux de tonalité pilote multicanaux via de multiples bobines d'émission (114), dans lequel les multiples canaux de réception sont configurés pour recevoir des données de tonalité pilote multicanaux via de multiples bobines de réception (116), dans lequel le procédé comprend :
- l'émission (202) des multiples signaux de tonalité pilote multicanaux par commande des multiples canaux d'émission ;
- l'acquisition (204) des données de tonalité pilote multicanaux par commande des multiples canaux de réception pour recevoir les données de tonalité pilote multicanaux ; et
- la détermination (206) d'un état de mouvement (140, 700, 700', 702, 702') du sujet en utilisant les données de tonalité pilote multicanaux.
